# EUROPEAN PATENT APPLICATION

(11) **EP 4 631 618 A1**
(43) Date of publication of application: **15.10.2025**
(21) Application number: 23900210.8
(22) Date of filing: 23.03.2023
(51) Int. Cl.: B01J 23/44, C07C 1/207, C07C 15/16, C07C 29/132, C07C 31/125, C07C 33/24, C07C 37/055, C07C 39/08, C07C 51/09, C07C 57/30, C07C 67/303, C07C 69/612, C07C 209/36, C07C 209/62, C07C 209/70, C07C 211/17, C07C 211/47, C07B 61/00

(54) **CERAMIC-LOADED PALLADIUM CATALYST**

(30) Priority: 06.12.2022 WO PCT/JP2022/044967
(71) Applicant: Sajiki, Hironao, Gifu-shi, Gifu 501-1196 (JP); Ikawa, Takashi, Gifu-shi, Gifu 501-1196 (JP); Yamada, Tsuyoshi, Gifu-shi, Gifu 501-1196 (JP); NGK Insulators, Ltd., Nagoya-shi, Aichi 467-8530 (JP)
(72) Inventor: YOKOYAMA Takashi, Nagoya-shi, Aichi 467-8530 (JP); NIWA Kosuke, Nagoya-shi, Aichi 467-8530 (JP); SAJIKI Hironao, Gifu-shi, Gifu 501-1196 (JP); IKAWA Takashi, Gifu-shi, Gifu 501-1196 (JP); YAMADA Tsuyoshi, Gifu-shi, Gifu 501-1196 (JP)
(74) Representative: Mewburn Ellis LLP
(86) International application number: PCT/JP2023/011549
(87) International publication number: WO 2024/122077

(57) **Abstract**

Provided are a ceramic-supported palladium catalyst, which has an excellent selective reduction property and is applicable to flow synthesis, a method of producing the ceramic-supported palladium catalyst, and a flow reaction apparatus including a ceramic-supported palladium catalyst. The ceramic-supported palladium catalyst according to an embodiment of the present disclosure includes: palladium serving as an active component; and a ceramics carrier for supporting the palladium. In the ceramics carrier, a content ratio of aluminum oxide is from 15 mass% to 45 mass%, a content ratio of silicon oxide is from 40 mass% to 60 mass%, and a content ratio of magnesium oxide is from 5 mass% to 30 mass%.

## Description

### Technical Field

The present disclosure relates to a ceramic-supported palladium catalyst, a method of producing a ceramic-supported palladium catalyst, and a flow reaction apparatus.

### Background Art

The production of various industrial products such as a pharmaceutical by organic synthesis has been known. In such organic synthesis, it has been investigated that an efficient synthesis scheme is established through use of a selective catalyst that causes a specific functional group to selectively react.

As the selective catalyst, there is a proposal of, for example, a catalyst for reduction, including a ceramic and palladium supported on the ceramic, wherein the ceramic contains 30 wt% to 45 wt% of silicon oxide, 25 wt% to 40 wt% of aluminum oxide, and 15 wt% to 30 wt% of calcium oxide (see, for example, Patent Literature 1).

In this technical field, in order to achieve a wide variety of organic synthesis, there has been an earnest desire for the development of novel catalysts having different selective reduction properties. In addition, hitherto, it has been considered that reaction conditions need to be precisely controlled in organic synthesis using a selective reduction catalyst, and hence the organic synthesis has been performed by a batch method. However, in recent years, organic synthesis by a flow method (flow synthesis) has attracted attention because of its energy productivity more excellent than that of the batch method, and hence the application of the selective reduction catalyst to the flow synthesis has been expected.

### Citation List

### Patent Literature

[PTL 1] JP 2015-180494 A

### Summary of Invention

### Technical Problem

A primary object of the present disclosure is to provide a ceramic-supported palladium catalyst, which has an excellent selective reduction property and is applicable to flow synthesis, a method of producing the ceramic-supported palladium catalyst, and a flow reaction apparatus including a ceramic-supported palladium catalyst.

### Solution to Problem

[1] A ceramic-supported palladium catalyst according to an embodiment of the present disclosure includes: palladium serving as an active component; and a ceramics carrier for supporting the palladium. The ceramics carrier contains aluminum oxide, silicon oxide, and magnesium oxide. In the ceramics carrier, a content ratio of aluminum oxide is from 15 mass% to 45 mass%, a content ratio of silicon oxide is from 40 mass% to 60 mass%, and a content ratio of magnesium oxide is from 5 mass% to 30 mass%.
[2] The ceramic-supported palladium catalyst according to the above-mentioned item [1] may be capable of reducing at least one functional group selected from an alkynylene group, an alkenylene group, an alkynyl group, an alkenyl group, an azide group, a nitro group, a carbobenzoxy group serving as a protective group for an amino group, an aromatic aldehyde group, a trialkylsilyloxy group, an arylalkyloxy group bonded to carbonyl carbon, an oxyalkylene group bonded to aromatic carbon, an arylalkyloxy group bonded to aromatic carbon, a halogeno group bonded to aromatic carbon, and an aromatic carbonyl group.
[3] In the ceramic-supported palladium catalyst according to the above-mentioned item [1] or [2], the palladium may be dispersed and supported on a surface of the ceramics carrier in a particulate manner. The palladium may contain flat particles.
[4] A method of producing a ceramic-supported palladium catalyst according to another aspect of the present disclosure includes the steps of: preparing a palladium compound solution by dissolving a palladium compound in a solvent; adding a ceramics carrier containing aluminum oxide, silicon oxide, and magnesium oxide to the palladium compound solution, followed by stirring; and removing the solvent from the palladium compound solution to which the ceramics carrier is added. In the ceramics carrier, a content ratio of aluminum oxide is from 15 mass% to 45 mass%, a content ratio of silicon oxide is from 40 mass% to 60 mass%, and a content ratio of magnesium oxide is from 5 mass% to 30 mass%.
[5] In the method of producing a ceramic-supported palladium catalyst according to the above-mentioned item [4], the palladium compound may contain a palladium(0) complex containing zerovalent palladium.
[6] In the method of producing a ceramic-supported palladium catalyst according to the above-mentioned item [5], the palladium(0) complex may contain tris(dibenzylideneacetone)dipalladium and/or tetrakis(triphenylphosphine)palladium.
[7] In the method of producing a ceramic-supported palladium catalyst according to any one of the above-mentioned items [4] to [6], the palladium compound may be oxidized by heating before being dissolved in the solvent.
[8] A flow reaction apparatus according to still another aspect of the present disclosure includes the ceramic-supported palladium catalyst of any one of the above-mentioned items [1] to [3].
[9] The flow reaction apparatus according to the above-mentioned item [8] may further include a filler. The filler is mixed with the selective reduction catalyst. A specific gravity of the filler with respect to the ceramic-supported palladium catalyst may be from 0.5 to 2.0.
[10] In the flow reaction apparatus according to the above-mentioned item [9], the specific gravity of the filler with respect to the ceramic-supported palladium catalyst may be 1.0±0.1. The ceramic-supported palladium catalyst and the filler may have shapes identical to each other.

### Advantageous Effects of Invention

According to the embodiments of the present disclosure, the ceramic-supported palladium catalyst, which has an excellent selective reduction property and is applicable to flow synthesis, the method of producing the ceramic-supported palladium catalyst, and the flow reaction apparatus including a ceramic-supported palladium catalyst can be achieved.

### Brief Description of Drawings

FIG. 1 is a backscattered electron image (BED) photograph of a ceramic-supported palladium catalyst of Example 1.
FIG. 2 is another backscattered electron image (BED) photograph of the ceramic-supported palladium catalyst of Example 1.

### Description of Embodiments

### A. Outline of Ceramic-supported Palladium Catalyst

A ceramic-supported palladium catalyst according to one embodiment of the present disclosure is a heterogeneous catalyst, and is capable of selectively reducing a specific functional group (reduction target functional group). The ceramic-supported palladium catalyst includes palladium serving as an active component and a ceramics carrier for supporting the palladium. The ceramics carrier contains aluminum oxide, silicon oxide, and magnesium oxide. A content ratio of aluminum oxide in the ceramics carrier is from 15 mass% to 45 mass%. A content ratio of silicon oxide in the ceramics carrier is from 40 mass% to 60 mass%. A content ratio of magnesium oxide in the ceramics carrier is from 5 mass% to 30 mass%. With such configuration, the ceramic-supported palladium catalyst has an excellent selective reduction property and is applicable to flow synthesis because the palladium is supported on the ceramics carrier having the above-mentioned specific composition.

### A-1. Ceramics Carrier

The ceramics carrier is typically substantially free of calcium oxide. The phrase "substantially free" as used herein encompasses not only a case in which the ceramics carrier is completely free of calcium oxide but also a case in which the content ratio of calcium oxide in the ceramics carrier is 1 mass% or less. The content ratio of calcium oxide in the ceramics carrier is preferably 0 mass%.

The ceramics carrier more preferably contains only aluminum oxide, silicon oxide, and magnesium oxide as oxides. The ceramics carrier is particularly preferably cordierite (2MgO·2Al₂O₃·5SiO₂).

The ceramics carrier is typically particulate. In one embodiment, the ceramics carrier is prepared by mixing the above-mentioned oxides at the above-mentioned ratios, followed by firing and pulverization by any appropriate method.

### A-2. Palladium

The palladium to be supported on the ceramics carrier may have any appropriate configuration. The valence of the palladium is, for example, 0 or more and 6 or less, preferably 0 or more and 2 or less, more preferably 0. When the valence of the palladium falls within the above-mentioned ranges, the activity of the ceramic-supported palladium catalyst can be improved.

The palladium to be supported on the ceramics carrier may be in a metal state, or may be in a state of being incorporated in a palladium compound to be described later.

In one embodiment, the palladium is dispersed and supported on a surface of the ceramics carrier in a particulate manner. The amount of the palladium supported in the ceramic-supported palladium catalyst is, for example, 0.5 mass% or more, preferably 1.0 mass% or more, and is, for example, 10.0 mass% or less. The amount of the palladium supported may be measured by a mass change of a palladium source before and after the supporting. When the amount of the palladium supported falls within the above-mentioned ranges, an excellent selective reduction property can be stably exhibited in the ceramic-supported palladium catalyst.

In one embodiment, the palladium to be supported on the ceramics carrier in a particulate manner contains flat particles. With such configuration, an excellent selective reduction property can be further stably exhibited in the ceramic-supported palladium catalyst.

In addition, the palladium to be supported in a particulate manner preferably contains fine particles smaller than the flat particles in addition to the flat particles.

### A-3. Functional Group that can be selectively reduced (Reduction Target Functional Group)

In one embodiment, the ceramic-supported palladium catalyst is capable of selective reduction of at least one reduction target functional group and causes selective non-reduction of at least one reduction non-target functional group. The term "selective reduction" as used herein means that a conversion rate in a reduction reaction is, for example, 85% or more, or, for example, 90% or more, and the term "selective non-reduction" means that the conversion rate in the reduction reaction is, for example, 15% or less, or, for example, 10% or less.

Examples of the reduction target functional group that can be reduced by the ceramic-supported palladium catalyst include an alkynylene group, an alkenylene group, an alkynyl group, an alkenyl group, an azide group, a nitro group, a carbobenzoxy group serving as a protective group for an amino group, an aromatic aldehyde group, a trialkylsilyloxy group, an arylalkyloxy group bonded to carbonyl carbon, an alkylene group bonded to aromatic carbon, an arylalkyloxy group bonded to aromatic carbon, a halogeno group bonded to aromatic carbon, and an aromatic carbonyl group. An example of such reduction target functional group is the "functional group to be selectively reduced" described in JP 2015-180494 A, the description of which is incorporated herein by reference.

Examples of the alkynylene group include alkynylene groups described in paragraph [0022] of JP 2015-180494 A. Of those, an ethynylene group, a propynylene group, a butynylene group, a pentynylene group, and a hexynylene group are preferred, and an ethynylene group and a propynylene group are more preferred.

Examples of the alkenylene group include alkenylene groups described in paragraph [0023] of JP 2015-180494 A. Of those, a vinylene group (ethenylene group), a propenylene group, a butenylene group, and a pentenylene group are preferred, and an ethenylene group and a vinylene group (ethenylene group) are more preferred.

Examples of the alkynyl group include alkynyl groups described in paragraph [0024] of JP 2015-180494 A. Of those, an ethynyl group, a propynyl group, a butynyl group, a pentynyl group, a hexynyl group, a heptynyl group, and an octynyl group are preferred, and an ethynyl group, a propynyl group, a butynyl group, a pentynyl group, and a hexynyl group are more preferred.

Examples of the alkenyl group include alkenyl groups described in paragraph [0025] of JP 2015-180494 A. Of those, a vinyl group (ethenyl group), a propenyl group (allyl group), a butenyl group, a pentenyl group, a hexenyl group, a heptenyl group, an octenyl group, a methylvinyl group, a methylpropenyl group, a methylbutenyl group, a methylpentenyl group, a methylhexenyl group, a methylheptenyl group, and a methyloctenyl group are preferred, and a vinyl group (ethenyl group), a propenyl group (allyl group), a methylvinyl group (methylethenyl group), a hexenyl group, an octenyl group, and a methylheptenyl group are more preferred.

The carbobenzoxy group serving as a protective group for an amino group is a carbobenzoxy group (benzyloxycarbonyl group) bonded to a nitrogen atom, and has a structure represented by the general formula [20] of JP 2015-180494 A.

The aromatic aldehyde group is an aldehyde group bonded to an aromatic ring. Examples of the aromatic ring include aromatic rings described in paragraph [0028] of JP 2015-180494 A. Of those, a phenyl group and a naphthyl group are preferred, and a phenyl group is more preferred.

Examples of the trialkylsilyloxy group include trialkylsilyloxy groups described in paragraph [0029] of JP 2015-180494 A. Of those, a triethylsilyloxy group, a tri-n-propylsilyloxy group, and a tri-n-butylsilyloxy group are preferred, and a triethylsilyloxy group and a tri-n-propylsilyloxy group are more preferred.

The arylalkyloxy group bonded to carbonyl carbon is directly bonded to a carbon atom in a carbonyl group (carbonyl carbon), and includes: an aryl group; an alkylene group bonded to an aryl group; and an oxygen atom for linking an alkylene group and carbonyl carbon.

As the aryl group, for example, a phenyl group and a naphthyl group are preferred, and a phenyl group is more preferred.

Examples of the alkylene group include alkylene groups each having 1 to 8 carbon atoms. Of those, alkylene groups each having 1 to 4 carbon atoms are more preferred, and a methylene group is still more preferred.

The arylalkyloxy group is particularly preferably a benzyloxy group.

The oxyalkylene group bonded to aromatic carbon is an oxyalkylene group bonded to the above-mentioned aromatic ring. Examples of the oxyalkylene group include an oxyethylene group and an oxypropylene group. Of those, an oxyethylene group is preferred. The aromatic ring is preferably a phenyl group.

The arylalkyloxy group bonded to aromatic carbon is an arylalkyloxy group bonded to the above-mentioned aromatic ring, and is preferably, for example, a benzyloxy group. The aromatic ring is preferably, for example, a phenyl group.

Examples of the halogeno group bonded to aromatic carbon is a halogen atom bonded to the above-mentioned aromatic ring. Examples of the halogen atom include fluorine, chlorine, bromine, and iodine. Of those, chlorine is preferred. The aromatic ring is preferably, for example, a phenyl group.

The aromatic carbonyl group is a carbonyl group bonded to the above-mentioned aromatic ring. The aromatic ring is preferably, for example, a phenyl group.

### A-4. Functional Group subjected to Selective Non-reduction (Reduction Non-target Functional Group)

Examples of the reduction non-target functional group include an alkyloxy group (alkoxy group), an alkyloxycarbonyl group (alkoxycarbonyl group), an alkylcarbonyl group, a halogeno group, a hydroxy group, a hydroxyalkyl group, an arylalkyloxy group bonded to a carbon atom in an aromatic ring (aromatic carbon), an arylalkyloxy group bonded to a carbon atom in an aliphatic hydrocarbon group (aliphatic carbon), a tert-butyldimethylsilyloxy group, and a triisopropylsilyloxy group.

Examples of the alkyloxy group (alkoxy group) include alkoxy groups described in paragraph [0032] of JP 2015-180494 A. Of those, a methoxy group, an ethoxy group, a n-propoxy group, and an isopropoxy group are preferred, and a methoxy group is more preferred.

Examples of the alkyloxycarbonyl group (alkoxycarbonyl group) include alkoxycarbonyl groups described in paragraph [0033] of JP 2015-180494 A. Of those, a methoxycarbonyl group, an ethoxycarbonyl group, a n-propoxycarbonyl group, and an isopropoxycarbonyl group are preferred, and a methoxycarbonyl group and an ethoxycarbonyl group are more preferred.

Examples of the alkylcarbonyl group include alkylcarbonyl groups described in paragraph [0034] of JP 2015-180494 A. Of those, a methylcarbonyl group, an ethylcarbonyl group, a n-propylcarbonyl group, an isopropylcarbonyl group, a n-butylcarbonyl group, an isobutylcarbonyl group, a sec-butylcarbonyl group, a tert-butylcarbonyl group, a n-pentylcarbonyl group, an isopentylcarbonyl group, a sec-pentylcarbonyl group, a tert-pentylcarbonyl group, a neopentylcarbonyl group, a n-hexylcarbonyl group, an isohexylcarbonyl group, a sec-hexylcarbonyl group, and a tert-hexylcarbonyl group are preferred, a methylcarbonyl group, an ethylcarbonyl group, a n-propylcarbonyl group, and an isopropylcarbonyl group are more preferred, and a methylcarbonyl group is still more preferred.

The halogeno group serving as the reduction non-target functional group is typically bonded to aliphatic carbon. Examples of the halogeno group include fluorine, chlorine, bromine, and iodine. Of those, chlorine and iodine are preferred.

Examples of the hydroxyalkyl group include hydroxyalkyl groups described in paragraph [0035] of JP 2015-180494 A. Of those, a hydroxymethyl group, a hydroxyethyl group, a hydroxyisopropyl group, and a hydroxy-n-propyl group are preferred, and a hydroxymethyl group is more preferred.

The arylalkyloxy group serving as the reduction non-target functional group is typically bonded to aliphatic carbon. Examples of the arylalkyloxy group bonded to aromatic carbon or aliphatic carbon include the above-mentioned arylalkyloxy groups. Of those, a benzyloxy group is preferred.

In one embodiment, the ceramic-supported palladium catalyst is capable of selective reduction of all of the above-mentioned reduction target functional groups, and causes selective non-reduction of the above-mentioned reduction non-target functional groups.

In addition, as long as the ceramic-supported palladium catalyst is capable of selective reduction of at least one functional group out of the above-mentioned reduction target functional groups, the other functional groups may be reduction non-target functional groups subjected to selective non-reduction.

In one embodiment, the ceramic-supported palladium catalyst is capable of reducing an alkynylene group, an alkenylene group, an alkynyl group, an alkenyl group, and an azide group out of the above-mentioned reduction target functional groups, and causes selective non-reduction of a nitro group, a carbobenzoxy group serving as a protective group for an amino group, an aromatic aldehyde group, and an arylalkyloxy group bonded to carbonyl carbon in addition to the above-mentioned reduction non-target functional groups.

In another embodiment, the ceramic-supported palladium catalyst is capable of selective reduction of an alkynylene group, an alkenylene group, an alkynyl group, an alkenyl group, a nitro group, an oxyalkylene group bonded to aromatic carbon, a carbobenzoxy group, an arylalkyloxy group bonded to aromatic carbon, an arylalkyloxy group bonded to carbonyl carbon, an aromatic carbonyl group, a halogeno group bonded to aromatic carbon, and an azide group out of the above-mentioned reduction target functional groups, and typically causes selective non-reduction of an arylalkyloxy group bonded to aliphatic carbon, an alkoxy group, an alkylcarbonyl group, and an oxyalkylene group bonded to aliphatic carbon out of the above-mentioned reduction non-target functional groups.

### B. Method of producing Ceramic-supported Palladium Catalyst

Next, a method of producing a ceramic-supported palladium catalyst is described. In one embodiment, the method of producing a ceramic-supported palladium catalyst includes the steps of: preparing a palladium compound solution (solution preparation step); adding a ceramics carrier to the palladium compound solution, followed by stirring (supporting step); and removing a solvent from the palladium compound solution (solvent removal step).

### B-1. Solution Preparation Step

In the solution preparation step, the palladium compound solution is prepared by dissolving a palladium compound in a solvent.

Examples of the palladium compound include a palladium(IV) compound containing tetravalent palladium, a palladium(II) compound containing divalent palladium, and a palladium(0) complex containing zerovalent palladium.

Examples of the palladium(IV) compound include hexachloropalladic acid, ammonium hexachloropalladate, sodium hexachloropalladate, and potassium hexachloropalladate.

Examples of the palladium(II) compound include palladium oxide, palladium sulfide, palladium chloride, palladium bromide, palladium iodide, palladium hydroxide, palladium nitrate, palladium sulfate, palladium cyanide, palladium acetate, bis(acetylacetonato)palladium, diamminedichloropalladium, diamminedinitropalladium, tetraamminepalladium chloride, tetraamminepalladium bromide, tetraamminepalladium nitrate, tetrachloropalladic acid, ammonium tetrachloropalladate, sodium tetrachloropalladate, potassium tetrachloropalladate, lithium tetrachloropalladate, potassium tetracyanopalladate, potassium tetrathiocyanatopalladate, bis(acetonitrile)dichloropalladium, bis(triphenylphosphine)palladium dichloride, and palladium trifluoroacetate.

Examples of the palladium(0) complex include tris(dibenzylideneacetone)dipalladium and tetrakis(triphenylphosphine)palladium.

Those palladium compounds may be used alone or in combination thereof.

Of the palladium compounds, for example, a palladium(II) compound and a palladium(0) complex are preferred, and bis(triphenylphosphine)palladium dichloride, palladium nitrate, palladium acetate, tris(dibenzylideneacetone)dipalladium, and tetrakis(triphenylphosphine)palladium are more preferred, and tris(dibenzylideneacetone)dipalladium and/or tetrakis(triphenylphosphine)palladium is still more preferred. When the palladium compound contains a palladium(II) compound and/or a palladium(0) complex, a ceramic-supported palladium catalyst can be stably produced.

In one embodiment, the above-mentioned palladium compound is oxidized by heating before being dissolved in a solvent. When the palladium compound is heated in advance, the palladium can be stably supported on the ceramics carrier. In other words, supporting reproducibility can be improved. In addition, the selective reduction property of the ceramic-supported palladium catalyst can be adjusted.

The palladium compound is typically heated under an oxidizing atmosphere (typically, air).

The heating temperature and heating time of the palladium compound may be adjusted to any appropriate temperature and time in accordance with the palladium compound to be heated. The heating temperature of the palladium compound is, for example, from 50°C to 100°C, preferably from 70°C to 90°C.

The heating time of the palladium compound is, for example, from 1 hour to 24 hours, preferably from 10 hours to 20 hours.

When the heating temperature and/or heating time of the palladium compound falls within such ranges, the supporting reproducibility can be stably improved.

Any appropriate solvent capable of dissolving the palladium compound may be adopted as the solvent. The solvent is typically an organic solvent. Examples of the organic solvent include: halogenated aliphatic hydrocarbons, such as chloroform, dichloromethane, and carbon tetrachloride; alcohols, such as methanol, ethanol, and isopropanol; esters, such as methyl acetate, ethyl acetate, and butyl acetate; ethers, such as diethyl ether and dioxane; nitriles such as acetonitrile; and polar aprotic solvents, such as dimethyl sulfoxide and dimethylacetamide.

Those solvents may be used alone or in combination thereof. Of the solvents, for example, halogenated aliphatic hydrocarbons, alcohols, esters, and polar aprotic solvents are preferred.

A palladium compound concentration in the palladium compound solution is, for example, 0.01 mass% or more, preferably 0.80 mass% or more, more preferably 0.15 mass% or more, and is, for example, 2.0 mass% or less, preferably 0.5 mass% or less.

### B-2. Supporting Step

Next, the above-mentioned ceramics carrier is added to the palladium compound solution, followed by stirring. More specifically, the palladium compound solution to which the ceramics carrier is added is stirred under an inert gas (e.g., nitrogen or argon) atmosphere.

The addition amount of the ceramics carrier is, for example, 5 parts by mass or more, preferably 20 parts by mass or more, and is, for example, 80 parts by mass or less, preferably 50 parts by mass or less with respect to 1 part by mass of palladium atoms in the palladium compound.

The addition amount of the ceramics carrier is, for example, 300 parts by mass or more, preferably 3,000 parts by mass or more, and is, for example, 50,000 parts by mass or less, preferably 10,000 parts by mass or less with respect to 100 parts by mass of the palladium compound solution.

A stirring temperature is, for example, 10°C or more and 40°C or less, and a stirring time is, for example, 0.5 hour or more, preferably 12 hours or more, and is, for example, 120 hours or less, preferably 48 hours or less.

Thus, a dispersion liquid in which the palladium compound is supported on the ceramics carrier, and in which the ceramics carrier for supporting the palladium compound is dispersed in the solvent is prepared.

### B-3. Solvent Removal Step

Next, the solvent is removed from the dispersion liquid by any appropriate method. Examples of the method of removing the solvent include filtration and evaporation under reduced pressure. Of those, filtration is preferred. When a filtrate and a residue (solid content) are separated by filtration, an impurity or the like dissolved in the solvent can be collectively separated from the residue (solid content).

Thus, the solid content in the dispersion liquid is recovered. When the valence of the palladium in the palladium compound is 2 or more, the resultant solid content is preferably subjected to reducing treatment with any appropriate reducing agent (e.g., a hydrazine aqueous solution). Thus, the palladium having a valence of 2 or more can be reduced to palladium(0).

After that, the solid content is washed as required, followed by drying.

Accordingly, a ceramic-supported palladium catalyst including the palladium and the ceramics carrier for supporting the palladium is produced.

### C. Flow Reaction Apparatus

The ceramic-supported palladium catalyst described in each of the above-mentioned sections A and B is applicable to any one of organic synthesis by a batch method (batch synthesis) and organic synthesis by a flow method (flow synthesis), and in particular, may be suitably adopted for the flow synthesis.

In one embodiment, a flow reaction apparatus capable of performing the flow synthesis includes the ceramic-supported palladium catalyst. The ceramic-supported palladium catalyst is typically loaded into a column in the flow reaction apparatus to form a fixed-bed catalyst.

In addition, the flow reaction apparatus preferably further includes a filler to be mixed with the ceramic-supported palladium catalyst. In this case, the ceramic-supported palladium catalyst is loaded into the column in a state of being mixed into the filler, and is uniformly dispersed in the column.

A specific gravity of the filler with respect to the ceramic-supported palladium catalyst (density of filler/density of ceramic-supported palladium catalyst) is, for example, from 0.1 to 3.0, preferably from 0.5 to 2.0, more preferably 1.0±0.1.

When the specific gravity of the filler with respect to the ceramic-supported palladium catalyst falls within the above-mentioned ranges, the ceramic-supported palladium catalyst can be stably fixed in a fixed bed, and hence, in the flow synthesis, the ceramic-supported palladium catalyst can be suppressed from flowing in the filler. Accordingly, the durability of the flow reaction apparatus can be improved.

In one embodiment, the ceramic-supported palladium catalyst and the filler have shapes identical to each other. An average primary particle diameter of the filler with respect to an average primary particle diameter of the ceramic-supported palladium catalyst (average primary particle diameter of filler/average primary particle diameter of ceramic-supported palladium catalyst) is, for example, from 0.1 to 3.0, preferably from 0.5 to 2.0, more preferably 1.0±0.1. The filler is preferably ceramics powder having the same composition as that of the ceramics carrier in the ceramic-supported palladium catalyst.

The mixing ratio of the filler is, for example, 300 parts by mass or more, preferably 800 parts by mass or more, and is, for example, 2,000 parts by mass or less, preferably 1,200 parts by mass or less with respect to 100 parts by mass of the ceramic-supported palladium catalyst.

In such flow reaction apparatus, a reactive substrate having at least the above-mentioned reduction target functional group can be continuously reduced. The reactive substrate preferably has a reduction non-target functional group in addition to the reduction target functional group. The reactive substrate is not particularly limited. Examples of the reactive substrate include compounds described in paragraph of [0038] to paragraph [0063] of JP 2015-180494 A.

In the continuous reduction reaction with the flow reaction apparatus, a reaction liquid obtained by dissolving the reactive substrate in any appropriate solvent is continuously supplied to the column. At this time, a hydrogen source (typically, hydrogen gas) is typically continuously supplied to the column together with the reaction liquid. Accordingly, when the reaction liquid passes through the column, hydrogen is donated from the hydrogen source to the reduction target functional group in the presence of the ceramic-supported palladium catalyst to reduce the reduction target functional group. Thus, a desired target compound can be smoothly produced.

### Examples

The present disclosure is specifically described below by way of Examples, but the present disclosure is not limited by these Examples.

### <Ceramic-supported Palladium Catalyst>

### <<Example 1>>

A palladium compound solution was prepared by dissolving 100 parts by mass of tetrakis(triphenylphosphine)palladium(0) serving as a palladium compound in 100,000 parts by mass of chloroform serving as an organic solvent. A combination of the palladium compound and the organic solvent is shown in Table 1.

Next, cordierite powder (ceramics carrier, 2MgO·2Al₂O₃·5SiO₂, average primary particle diameter: 80 µm) was added to the palladium compound solution at a ratio of 100 parts by mass with respect to 3 parts by mass of palladium atoms in the palladium compound, followed by replacement with an argon gas atmosphere, and the contents were stirred and mixed at normal temperature (23°C) for 24 hours. Thus, a dispersion liquid in which palladium(0) was supported on the cordierite powder and the cordierite powder supporting palladium(0) was dispersed in the organic solvent was prepared.

Next, the dispersion liquid was separated into a filtrate and a residue (solid content) by suction filtration. After that, the residue was washed with water, and then dried under reduced pressure at normal temperature (23°C) for 24 hours.

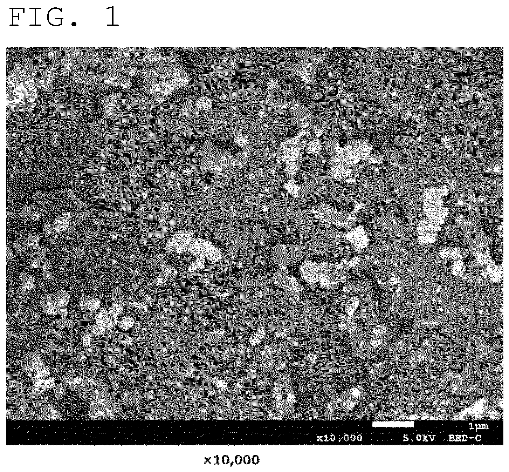

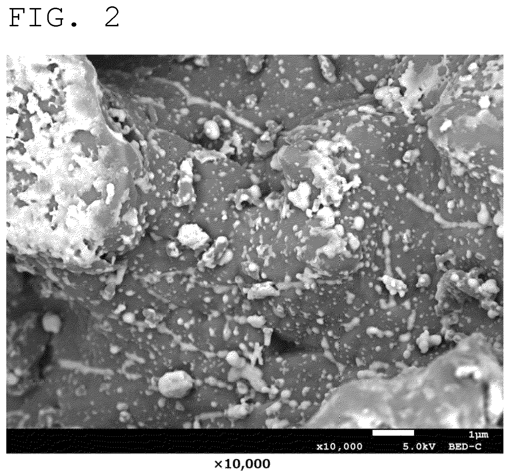

Thus, a powdery ceramic-supported palladium catalyst was obtained. Backscattered electron image (BED) photographs of the ceramic-supported palladium catalyst of Example 1 were taken with a scanning electron microscope including a backscattered electron detector. The backscattered electron image (BED) photographs of the ceramic-supported palladium catalyst are shown in FIG. **1** and FIG. **2****.**

In the BED photograph of the ceramic-supported palladium catalyst, it is observed that palladium (relatively white part) is dispersed and supported on a surface of a cordierite carrier (ceramics carrier, relatively black part) in a particulate manner. The palladium contained relatively large flat particles and relatively small fine particles.

In addition, the amount of the palladium compound in the filtrate was measured with an atomic absorption spectrometer (manufactured by Shimadzu Corporation), and the amount of the palladium supported on the cordierite carrier was calculated by subtracting the amount of the palladium compound in the filtrate from the load amount of the palladium compound. The amount of the palladium supported was 3 mass%.

### <<Example 2>>

A powdery ceramic-supported palladium catalyst was obtained in the same manner as in Example 1 except that 100,000 parts by mass of chloroform was changed to 53,000 parts by mass of ethanol. The amount of the palladium supported in the ceramic-supported palladium catalyst was 3 mass%.

### <<Example 3>>

A powdery ceramic-supported palladium catalyst was obtained in the same manner as in Example 1 except that 100 parts by mass of tetrakis(triphenylphosphine)palladium(0) was changed to 100 parts by mass of tris(dibenzylideneacetone)dipalladium(0). The amount of the palladium supported in the ceramic-supported palladium catalyst was 3 mass%.

### <<Example 4>>

A powdery ceramic-supported palladium catalyst was obtained in the same manner as in Example 3 except that 100,000 parts by mass of chloroform was changed to 53,000 parts by mass of ethanol. The amount of the palladium supported in the ceramic-supported palladium catalyst was 3 mass%.

### <<Example 5>>

A palladium compound solution was prepared by dissolving 100 parts by mass of bis(triphenylphosphine)palladium(II) dichloride serving as a palladium compound in 73,000 parts by mass of dimethyl sulfoxide serving as an organic solvent. Next, cordierite powder (ceramics carrier, 2MgO·2Al₂O₃·5SiO₂, average primary particle diameter: 80 µm) was added to the palladium compound solution at a ratio of 100 parts by mass with respect to 3 parts by mass of palladium atoms in the palladium compound, followed by replacement with an argon gas atmosphere, and the contents were stirred and mixed at normal temperature (23°C) for 24 hours. Thus, a dispersion liquid in which palladium(II) was supported on the cordierite powder and the cordierite powder supporting palladium(II) was dispersed in the organic solvent was prepared.

Next, the dispersion liquid was separated into a filtrate and a residue (solid content) by suction filtration. After that, the residue was added to a hydrazine aqueous solution, followed by stirring. Thus, palladium(II) was reduced to palladium(0). After that, the residue was washed with water, and then dried under reduced pressure at normal temperature (23°C) for 24 hours.

Thus, a powdery ceramic-supported palladium catalyst was obtained. In addition, the amount of the palladium supported was calculated in the same manner as in Example 1. The amount of the palladium supported was 3 mass%.

### <<Example 6>>

A powdery ceramic-supported palladium catalyst was obtained in the same manner as in Example 5 except that 73,000 parts by mass of dimethyl sulfoxide was changed to 60,000 parts by mass of dimethylacetamide. The amount of the palladium supported in the ceramic-supported palladium catalyst was 3 mass%.

### <<Example 7>>

A powdery ceramic-supported palladium catalyst was obtained in the same manner as in Example 5 except that: 100 parts by mass of bis(triphenylphosphine)palladium(II) dichloride was changed to 100 parts by mass of palladium(II) nitrate; and 73,000 parts by mass of dimethyl sulfoxide was changed to 53,000 parts by mass of acetonitrile. The amount of the palladium supported in the ceramic-supported palladium catalyst was 3 mass%.

### <<Example 8>>

A powdery ceramic-supported palladium catalyst was obtained in the same manner as in Example 5 except that: 100 parts by mass of bis(triphenylphosphine)palladium(II) dichloride was changed to 100 parts by mass of palladium(II) acetate; and 73,000 parts by mass of dimethyl sulfoxide was changed to 53,000 parts by mass of methanol. The amount of the palladium supported in the ceramic-supported palladium catalyst was 3 mass%.

### <<Example 9>>

A powdery ceramic-supported palladium catalyst was obtained in the same manner as in Example 8 except that 53,000 parts by mass of methanol was changed to 53,000 parts by mass of acetonitrile. The amount of the palladium supported in the ceramic-supported palladium catalyst was 4.8 mass%.

### <<Example 10>>

A powdery ceramic-supported palladium catalyst was obtained in the same manner as in Example 8 except that 53,000 parts by mass of methanol was changed to 60,000 parts by mass of ethyl acetate. The amount of the palladium supported in the ceramic-supported palladium catalyst was 4.8 mass%.

### <Flow Reaction; Flow Reaction Apparatus>

### <<Example 11>>

50 Milligrams of the ceramic-supported palladium catalyst of Example 1 and 500 mg of cordierite powder (filler, 2MgO·2Al₂O₃·5SiO₂, average primary particle diameter: 80 µm) were mixed to provide mixed powder. Next, the mixed powder was loaded into a column (diameter 5 mm×length 50 mm, stainless steel-made). A specific gravity of the filler with respect to the ceramic-supported palladium catalyst (density of filler/density of ceramic-supported palladium catalyst) was about 1, and the ceramic-supported palladium catalyst and the filler had shapes identical to each other.

Thus, a flow reaction apparatus including the column having the mixed powder loaded thereinto was prepared.

In addition, a reactive substrate shown in Table 2 was dissolved in methanol to prepare a 0.1 mol/L reaction liquid.

Next, a column temperature was adjusted to 50°C, and then the reaction liquid was supplied to the column at a flow rate of **0.1** mL/min, and hydrogen gas was continuously supplied thereto at a flow rate of 2.0 mL/min.

After that, the reaction liquid having passed through the column was recovered, and the solvent was removed from the reaction liquid. Thus, a solid content containing the reactive substrate and/or a reaction product was obtained. Next, the solid content was dissolved in deuterated chloroform, and the reactive substrate and the reaction product were analyzed with a nuclear magnetic resonance apparatus (¹H-NMR). The reactive substrate and the reaction product, and peak area ratios of the NMR spectra thereof are shown in Table 2.

In addition, a selective reduction property of the ceramic-supported palladium catalyst was evaluated by the following criteria with the ratio of the peak area of the reaction product with respect to the total of the peak areas of the reactive substrate and the reaction product. The results are shown in Table 1.
R (selective reduction): The ratio of the peak area of the reaction product was 90% or more.
NR (selective non-reduction): The ratio of the peak area of the reaction product was 10% or less.
× (low selectivity): The ratio of the peak area of the reaction product was more than 10% and less than 90%.

### <Batch Reaction>

### <<Examples 12 to 20>>

9 Milligrams of each of the ceramic-supported palladium catalysts of Examples 2 to 10, 1 mL of methanol, and 38 mg of 1-ethyl-4-nitrobenzene shown in Table 2 were loaded into a test tube, followed by replacement of the inside of the test tube with a hydrogen atmosphere. Next, an inner temperature of the test tube was adjusted to 25°C, and a reaction was performed for 24 hours.

After that, the reaction liquid in the test tube was recovered, and a reactive substrate and/or a reaction product was recovered and analyzed, and was evaluated by the above-mentioned criteria. The results are shown in Table 1.

In each of the ceramic-supported palladium catalysts of Examples 2 to 7, 9, and 10, a nitro group was selectively nonreduced. In those ceramic-supported palladium catalysts, it was expected that a functional group that was more hardly reduced than the nitro group was unable to be reduced, and hence the experiment on the reduction with a flow reaction apparatus was not performed.

Meanwhile, in the ceramic-supported palladium catalyst of Example 8, a nitro group was selectively reduced, but it was expected that the ceramic-supported palladium catalyst showed the same tendency as in Example 1. Accordingly, the experiment on the reduction with the flow reaction apparatus was not performed.

**Table 1**

| No. | | | | Example 1 | Example 2 | | Example 3 |
|---|---|---|---|---|---|---|---|
| Palladium compound | | | | (PPh₃)₄Pd | (PPh₃)₄Pd | | Pd₂(dba)₃ |
| Organic solvent | | | | CHCl₃ | EtOH | | CHCl₃ |
| | | | | Example 11 | Example 12 | | Example 13 |
| | | | | Flow reaction | Batch r eaction | | |
| Reducibility | Nitro group | | | R | NR | | NR |
| | Bonding target | Carbonyl carbon | AAO group* | R | | | |
| | | Aromatic carbon | | NR | | | |
| | | Aliphatic carbon | | × | | | |
| | Hydroxy group | | | NR | | | |
| | Alkenylene group | | | R | | | |
| | Alkenyl group | | | R | | | |
| | Carbobenzoxy group | | | R | | | |
| | Aromatic carbonyl group | | | × | | | |

| No. | | | | Example 4 | | Example 5 | |
|---|---|---|---|---|---|---|---|
| Palladium compound | | | | Pd₂(dba)₃ | | (PPh₃)₂PdCl₂ | |
| Organic solvent | | | | EtOH | | DMSO | |
| | | | | Example 14 | | Example 15 | |
| | | | | Batch reaction | | | |
| Reducibility | Nitro group | | | NR | | NR | |
| | Bonding target | Carbonyl carbon | AAO group* | | | | |
| | | Aromatic carbon | | | | | |
| | | Aliphatic carbon | | | | | |
| | Hydroxy group | | | | | | |
| | Alkenylene group | | | | | | |
| | Alkenyl group | | | | | | |
| | Carbobenzoxy group | | | | | | |
| | Aromatic carbonyl group | | | | | | |

| No. | | | | Example 6 | | Example 7 | |
|---|---|---|---|---|---|---|---|
| Palladium compound | | | | (PPh₃)₂PdCl₂ | | Pd(NO₃)₂ | |
| Organic solvent | | | | DMA | | MeCN | |
| | | | | Example 16 | | Example 17 | |
| | | | | Batch reaction | | | |
| Reducibility | Nitro group | | | NR | | NR | |
| | Bonding target | Carbonyl carbon | AAO group* | | | | |
| | | Aromatic carbon | | | | | |
| | | Aliphatic carbon | | | | | |
| | Hydroxy group | | | | | | |
| | Alkenylene group | | | | | | |
| | Alkenyl group | | | | | | |
| | Carbobenzoxy group | | | | | | |
| | Aromatic carbonyl group | | | | | | |

| No. | | | | Example 8 | Example 9 | | Example 10 |
|---|---|---|---|---|---|---|---|
| Palladium compound | | | | Pd(OAc)₂ | Pd(OAc)₂ | | Pd(OAc)₂ |
| Organic solvent | | | | MeOH | MeCN | | AcOEt |
| | | | | Example 18 | Example 19 | | Example 20 |
| | | | | Batch reaction | | | |
| Reducibility | Nitro group | | | R | NR | | NR |
| | Bonding target | Carbonyl carbon | AAO group* | | | | |
| | | Aromatic carbon | | | | | |
| | | Aliphatic carbon | | | | | |
| | Hydroxy group | | | | | | |
| | Alkenylene group | | | | | | |
| | Alkenyl group | | | | | | |
| | Carbobenzoxy group | | | | | | |
| | Aromatic carbonyl group | | | | | | |

| | | | | | | | |
|---|---|---|---|---|---|---|---|
| *Arylalkyloxy group | | | | | | | |

The details of the abbreviations in Table 1 are shown below.

### [Palladium Compound]

(PPh₃)₄Pd: tetrakis(triphenylphosphine)palladium(0)
Pd₂(dba)₃: tris(dibenzylideneacetone)dipalladium(0)
(PPh₃)₂PdCl₂: bis(triphenylphosphine)palladium(II) dichloride Pd(NO₃)₂: palladium(II) nitrate
Pd(OAc)₂: palladium(II) acetate

### [Organic Solvent]

CHCl₃: chloroform
EtOH: ethanol
MeOH: methanol
MeCN: acetonitrile
AcOEt: ethyl acetate
DMSO: dimethyl sulfoxide
DMA: dimethylacetamide

**Table 2**

| Target functional group | Reactive substrate | Reaction product | |
|---|---|---|---|
| Nitro group | 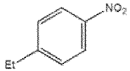 | 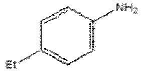 | |
| ¹H-NMR ratio [%] | 0 | 100 | |

| Target functional group | Reactive substrate | Reaction product | |
|---|---|---|---|
| Benzyloxy group (Aromatic carbon) Hydroxy group | 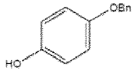 | 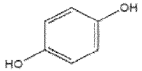 | |
| ¹H-NMR ratio [%] | 96 | 4 | |

| Target functional group | Reactive substrate | Reaction product | |
|---|---|---|---|
| Benzyloxy group (Aliphatic carbon) | 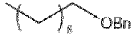 | 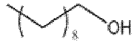 | |
| ¹H-NMR ratio [%] | 86 | 14 | |

| Target functional group | Reactive substrate | Reaction product | |
|---|---|---|---|
| Carbobenzoxy group | 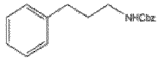 | 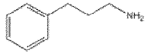 | |
| ¹H-NMR ratio [%] | 0 | 100 | |

| Target functional group | Reactive substrate | Reaction product | |
|---|---|---|---|
| Aromatic carbonyl group | 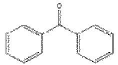 | 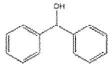 | 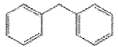 |
| ¹H-NMR ratio [%] | 56 | 39 | 5 |

| Target functional group | Reactive substrate | Reaction product | |
|---|---|---|---|
| Alkenylene group Benzyloxy group (Carbonyl carbon | 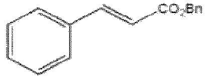 | 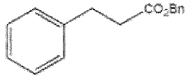 | 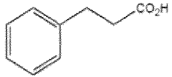 |
| ¹H-NMR ratio [%] | 0 | 4 | 96 |

| Target functional group | Reactive substrate | Reaction product | |
|---|---|---|---|
| Alkenyl group Carbobenzoxy group | 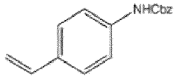 | 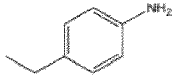 | |
| ¹H-NMR ratio [%] | 0 | 100 | |

### <Evaluation>

As shown in Table 1 and Table 2, it was observed that the ceramic-supported palladium catalyst of Example 1 was applicable to flow synthesis, and caused selective reduction of a nitro group, an arylalkyloxy group bonded to carbonyl carbon, an alkenylene group, an alkenyl group, and a carbobenzoquinone group, and selective non-reduction of an arylalkyloxy group bonded to an aromatic ring and a hydroxy group.

In addition, in each of the ceramic-supported palladium catalysts of Examples 2 to 7, 9, and 10, selective non-reduction of a nitro group was observed, and in the ceramic-supported palladium catalyst of Example 8, selective reduction of a nitro group was observed.

### <<Example 21>>

A powdery ceramic-supported palladium catalyst was obtained in the same manner as in Example 1 except that tetrakis(triphenylphosphine)palladium(0) serving as a palladium compound was heated in air at 80°C for 16 hours before being dissolved in chloroform serving as an organic solvent.

### <Flow Reaction; Flow Reaction Apparatus>

### <<Example 22>>

50 Milligrams of the ceramic-supported palladium catalyst of Example 21 and 500 mg of cordierite powder (filler, 2MgO·2Al₂O₃·5SiO₂, average primary particle diameter: 80 µm) were mixed to provide mixed powder. Next, the mixed powder was loaded into a column (diameter 5 mm×length 50 mm, stainless steel-made). A specific gravity of the filler with respect to the ceramic-supported palladium catalyst (density of filler/density of ceramic-supported palladium catalyst) was about 1, and the ceramic-supported palladium catalyst and the filler had shapes identical to each other.

Thus, a flow reaction apparatus including the column having the mixed powder loaded thereinto was prepared.

In addition, a reactive substrate shown in Table 3 and Table 4 was dissolved in methanol to prepare a 0.1 mol/L reaction liquid.

Next, a column temperature was adjusted to 50°C, and then the reaction liquid was supplied to the column at a flow rate of 0.1 mL/min, and hydrogen gas was continuously supplied thereto at a flow rate of 2.0 mL/min.

After that, the reaction liquid having passed through the column was recovered, and the solvent was removed from the reaction liquid. Thus, a solid content containing the reactive substrate and/or a reaction product was obtained. Next, the solid content was dissolved in deuterated chloroform, and the reactive substrate and the reaction product were analyzed with a nuclear magnetic resonance apparatus (¹H-NMR). The reactive substrate and the reaction product, and peak area ratios of the NMR spectra thereof are shown in Table 3 and Table 4.

**Table 3**

| Target functional group | Reactive substrate | Reaction product | |
|---|---|---|---|
| Nitro group | 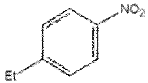 | 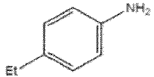 | |
| ¹H-NMR ratio [%] | 0 | 100 | |

| Target functional group | Reactive substrate | Reaction product | |
|---|---|---|---|
| Oxyethylene group (Aromatic carbon) | 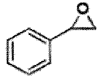 | 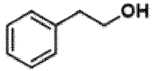 | |
| ¹H-NMR ratio [%] | 3 | 97 | |

| Target functional group | Reactive substrate | Reaction product | |
|---|---|---|---|
| Benzyloxy group (Aliphatic carbon) | 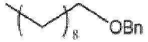 | 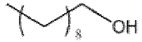 | |
| ¹H-NMR ratio [%] | 98 | 2 | |

| Target functional group | Reactive substrate | Reaction product | |
|---|---|---|---|
| Carbobenzoxy group | 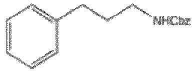 | 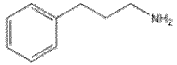 | |
| ¹H-NMR ratio [%] | 0 | 100 | |

| Target functional group | Reactive substrate | Reaction product | |
|---|---|---|---|
| Alkenylene group Benzyloxy group (Aromatic carbon) Alkoxy group | 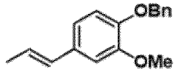 | 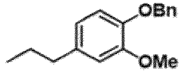 | 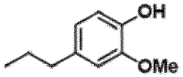 |
| ¹H-NMR ratio [%] | 0 | 0 | 100 |
| Alkenylene group Benzyloxy group (Carbonyl carbon) | 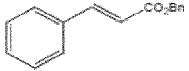 | 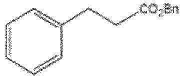 | 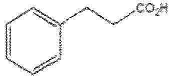 |
| ¹H-NMR ratio [%] | 0 | 0 | 100 |

| Target functional group | Reactive substrate | Reaction product | |
|---|---|---|---|
| Alkenyl group Carbobenzoxy group | 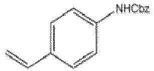 | 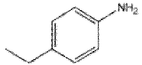 | |
| ¹H-NMR ratio [%] | 0 | 100 | |

| Target functional group | Reactive substrate | Reaction product | |
|---|---|---|---|
| Oxyethylene group (Aliphatic carbon) | 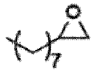 | 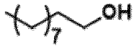 | |
| ¹H-NMR ratio [%] | 99 | 1 | |

**Table 4**

| Target functional group | Reactive substrate | Reaction product | |
|---|---|---|---|
| Azide group | 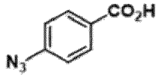 | 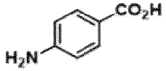 | |
| ¹H-NMR ratio [%] | 0 | 100 | |

| Target functional group | Reactive substrate | Reaction product | |
|---|---|---|---|
| Aromatic carbonyl group | 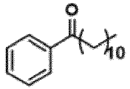 | 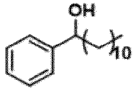 | |
| ¹H-NMR ratio [%] | 2 | 98 | |

| Target functional group | Reactive substrate | Reaction product | |
|---|---|---|---|
| Alkylcarbonyl group | 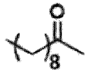 | 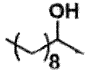 | |
| ¹H-NMR ratio [%] | 100 | 0 | |

| Target functional group | Reactive substrate | Reaction product | |
|---|---|---|---|
| Azide group Nitro group | 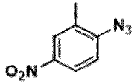 | 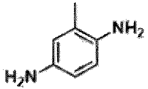 | |
| ¹H-NMR ratio [%] | 14 | 86 | |

| Target functional group | Reactive substrate | Reaction product | |
|---|---|---|---|
| Carbobenzoxy group | 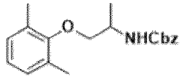 | 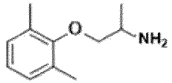 | |
| ¹H-NMR ratio [%] | 1 | 99 | |

| Target functional group | Reactive substrate | Reaction product | |
|---|---|---|---|
| Halogeno group (Aromatic carbon) | 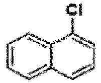 | 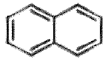 | |
| ¹H-NMR ratio [%] | 12 | 88 | |

### <Evaluation>

As shown in Table 3 and Table 4, it was observed that the ceramic-supported palladium catalyst of Example 21 was applicable to flow synthesis, was capable of selective reduction of an alkenylene group, an alkenyl group, a nitro group, an oxyethylene group bonded to aromatic carbon, a carbobenzoxy group, a benzyloxy group bonded to aromatic carbon, an aromatic carbonyl group, a benzyloxy group bonded to carbonyl carbon, an azide group, and a halogeno group bonded to aromatic carbon, and caused selective non-reduction of a benzyloxy group bonded to aliphatic carbon, an alkoxy group, an alkylcarbonyl group, and an oxyethylene group bonded to aliphatic carbon.

In this Example, it was judged that a selective reduction property was exhibited when the ratio of the peak area of the reaction product was 85% or more, and a selective non-reduction property was exhibited when the ratio of the peak area of the reaction product was 15% or less.

### Industrial Applicability

The ceramic-supported palladium catalyst according to the embodiment of the present disclosure is appropriately used for various industrial products utilizing palladium as a catalyst, and can be particularly suitably used as a reaction catalyst to be utilized in organic synthesis.

## Claims

1. A ceramic-supported palladium catalyst, comprising:
palladium serving as an active component; and
a ceramics carrier for supporting the palladium, the ceramics carrier containing aluminum oxide, silicon oxide, and magnesium oxide,
wherein, in the ceramics carrier, a content ratio of aluminum oxide is from 15 mass% to 45 mass%, a content ratio of silicon oxide is from 40 mass% to 60 mass%, and a content ratio of magnesium oxide is from 5 mass% to 30 mass%.

2. The ceramic-supported palladium catalyst according to claim 1, wherein the ceramic-supported palladium catalyst is capable of reducing at least one functional group selected from an alkynylene group, an alkenylene group, an alkynyl group, an alkenyl group, an azide group, a nitro group, a carbobenzoxy group serving as a protective group for an amino group, an aromatic aldehyde group, a trialkylsilyloxy group, an arylalkyloxy group bonded to carbonyl carbon, an oxyalkylene group bonded to aromatic carbon, an arylalkyloxy group bonded to aromatic carbon, a halogeno group bonded to aromatic carbon, and an aromatic carbonyl group.

3. The ceramic-supported palladium catalyst according to claim 1, wherein the palladium is dispersed and supported on a surface of the ceramics carrier in a particulate manner, and contains flat particles.

4. A method of producing a ceramic-supported palladium catalyst, comprising the steps of:
preparing a palladium compound solution by dissolving a palladium compound in a solvent;
adding a ceramics carrier containing aluminum oxide, silicon oxide, and magnesium oxide to the palladium compound solution, followed by stirring; and
removing the solvent from the palladium compound solution to which the ceramics carrier is added,
wherein, in the ceramics carrier, a content ratio of aluminum oxide is from 15 mass% to 45 mass%, a content ratio of silicon oxide is from 40 mass% to 60 mass%, and a content ratio of magnesium oxide is from 5 mass% to 30 mass%.

5. The method of producing a ceramic-supported palladium catalyst according to claim 4, wherein the palladium compound contains a palladium(0) complex containing zerovalent palladium.

6. The method of producing a ceramic-supported palladium catalyst according to claim 5, wherein the palladium(0) complex contains tris(dibenzylideneacetone)dipalladium and/or tetrakis(triphenylphosphine)palladium.

7. The method of producing a ceramic-supported palladium catalyst according to claim 4, wherein the palladium compound is oxidized by heating before being dissolved in the solvent.

8. A flow reaction apparatus, comprising the ceramic-supported palladium catalyst of any one of claims 1 to 3.

9. The flow reaction apparatus according to claim 8, further comprising a filler to be mixed with the selective reduction catalyst,
wherein a specific gravity of the filler with respect to the ceramic-supported palladium catalyst is from 0.5 to 2.0.

10. The flow reaction apparatus according to claim 9,
wherein the specific gravity of the filler with respect to the ceramic-supported palladium catalyst is 1.0±0.1, and
wherein the ceramic-supported palladium catalyst and the filler have shapes identical to each other.
